(19) Europäisches Patentamt
European Patent Office
Office européen des brevets

(11) EP 1 235 159 B1

(12) # EUROPEAN PATENT SPECIFICATION

(45) Date of publication and mention of the grant of the patent:
**14.02.2007 Bulletin 2007/07**

(21) Application number: **00976284.0**

(22) Date of filing: **16.11.2000**

(51) Int Cl.:
***G06F 17/30** (2006.01)*

(86) International application number:
**PCT/JP2000/008078**

(87) International publication number:
**WO 2001/039041 (31.05.2001 Gazette 2001/22)**

(54) **ID SYMBOL UNIQUE TO STRUCTURAL FORMULA OF COMPOUND**

IDENTIFIKATIONSSYMBOL DAS EINMALIG FÜR DIE STRUKTUR DER FORMEL EINER MISCHUNG IST

UNICITE DE SYMBOLIQUE D'IDENTIFICATION POUR FORMULE DE STRUCTURE DE COMPOSE

(84) Designated Contracting States:
**AT BE CH CY DE DK ES FI FR GB GR IE IT LI LU MC NL PT SE TR**

(30) Priority: **19.11.1999 JP 33043299**
**22.05.2000 JP 2000149641**

(43) Date of publication of application:
**28.08.2002 Bulletin 2002/35**

(73) Proprietor: **Institute of Medicinal Molecular Design, Inc.**
**Tokyo 113-0033 (JP)**

(72) Inventors:
- **TOYODA, Tetsuro**
  **Musashimurayama-shi**
  **Tokyo 208-0021 (JP)**
- **ITAI, Akiko**
  **Tokyo 113-0033 (JP)**

(74) Representative: **polypatent**
**Postfach 11 07**
**51482 Overath (DE)**

(56) References cited:
**EP-A- 0 829 810** **WO-A2-96/06391**

- **WIPKE W T ET AL: "Hash functions for rapid storage and retrieval of chemical structures" JOURNAL OF CHEMICAL INFORMATION AND COMPUTER SCIENCES, FEB. 1978, USA, vol. 18, no. 1, pages 32-37, XP002229554 ISSN: 0095-2338**
- **BELLARE M ET AL: "COLLISION-RESISTANT HASHING: TOWARDS MAKING UOWHFS PRACTICAL" ADVANCES IN CRYPTOLOGY - CRYPTO '97. SANTA BARBARA, AUG. 17 - 21, 1997, PROCEEDINGS OF THE ANNUAL INTERNATIONAL CRYPTOLOGY CONFERENCE (CRYPTO), BERLIN, SPRINGER, DE, vol. CONF. 17, 17 August 1997 (1997-08-17), pages 470-484, XP000767550 ISBN: 3-540-63384-7**
- **WIPKE W T ET AL: "STEREOCHEMICALLY UNIQUE NAMING ALGORITHM" JOURNAL OF THE AMERICAN CHEMICAL SOCIETY, AMERICAN CHEMICAL SOCIETY, WASHINGTON, DC, US, vol. 96, no. 15, 24 July 1974 (1974-07-24), pages 4834-4842, XP002935898 ISSN: 0002-7863**
- **IHLENFELDT W D ET AL: "HASH CODES FOR THE IDENTIFICATION AND CLASSIFICATION OF MOLECULAR STRUCTURE ELEMENTS" JOURNAL OF COMPUTATIONAL CHEMISTRY, JOHN WILEY AND SONS, CHICHESTER, GB, vol. 15, no. 8, 1994, pages 793-813, XP002935899 ISSN: 0192-8651**
- **WIPKE W T ET AL: 'Stereochemically unique naming algorithm' JOURNAL OF THE AMERICAN CHEMICAL SOCIETY vol. 96, no. 15, 24 July 1974, pages 4834 - 4872, XP002935898**
- **IHLENFELDT W.D. & GASTEIGER J.: 'Hash codes for the identification and classification of molecular structure elements' JOURNAL OF COMPUTATIONAL CHEMISTRY vol. 15, no. 8, August 1994, pages 793 - 813, XP002935899**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- FUKUDA ET AL.: 'Seibutsu johou tougou database system no kouchiku; Atarashii hassou ni motozuita seibutsu johou kanrihou' DAI 22KAI JOUHOU KAGAKU TOURONKAI, DAI 27 KAI KOUZOU KASSEI SOUKAN SYMPOSIUM KOUEN YOUSHISHU 31 October 1999, pages 84 - 85, XP002935900

## Description

### Technical Field

The present invention relates to a method of generating a character string of fixed or variable length which is substantially unique with respect to a chemical structural formula of a compound as an ID symbol of the compound or information related to the compound.

### Background Art

In recent years, screening of many compounds is carried out for the purpose of finding active compounds useful for drug development. Compounds on the market mount up to 1 million kinds at present. In pharmaceutical companies, these huge number of compounds are stored as libraries. Therefore, database preparation of huge compound information and useful method of searching compounds with the same chemical structure or similar chemical structure effectively are needed.

A compound is uniquely specified by a chemical structural formula indicating the kind of atoms constituting the compound and bonding relation of the atoms. On the other hand, for printing and publication purposes, methods of specifying a chemical structural formula with words or letters are necessary, and nomenclature of compounds has been studied since early times for that purpose. As a nomenclature, IUPAC method and Chemical Abstract method are well-known. However, the nomenclature beside these methods is still employed. For example, a conventional name that was arbitrarily given by a discoverer of a new compound is frequently used for natural compounds and others. Advanced skills are required for applying nomenclature rules strictly, however, ordinary organic chemists who use nomenclature are not skilled enough in nomenclature rules. Therefore, there are many cases in which different names are given depending on the person who applied nomenclature. Furthermore, even in the case wherein IUPAC nomenclature rule is conducted by a computer, there are problematic cases wherein naming is impossible for huge and complicated chemical structural formula or naming is not given uniquely. After all, since there is a possibility of existence of many ways of naming or designation for one compound, and since it is not possible to give a name of fixed length as a compound name by a formal nomenclature method which aims at precise description, the above-mentioned nomenclature methods are not suitable for management by computer.

Consequently, compound databases wherein searching is possible by using a chemical structural formula directly as a query, rather than by nomenclature, have become popular recently. Using a computer software, and by drawing a chemical structural formula on computer display, it is possible to input the kind of atoms and bonding relation between atoms. Search can be carried out using the said chemical structural formula as a query. As such a database specification and a search method, ISIS by MDL Inc. (U.S.A.) is well-known. By specifying the kind of atoms and the linkage between atoms, this method can be used conveniently for searching chemical structural formulas which include these as a partial structure. However, this method requires data on chemical structural formulas for comparison to be stored in the database, and a dedicated software for the comparison of chemical structural formulas is also necessary. Since a chemical structural formula is not character string data, it is not possible to search from a chemical structural formula utilizing an index search software.

Chemical Abstract, which is a database of compounds published on academic journals or filed for patent application, is well-known as a compound database based on nomenclature. On the other hand, ACD, which is a database of commercial compounds, is well-known as a compound database based on the chemical structural formula. In the latter database, an identifier (ID symbol) consisted of 6 to 10 alphanumeric characters is given to each data record of a compound. However, since those ID symbols are given irrelevant to chemical structures, there are many cases in which different ID symbols are given to the same structure. For this reason, it is not possible to confirm whether chemical structural formulas are the same or not from the said ID symbols like the structural formula and the compound name by nomenclature.

EP 0 829 810 A1 discloses a biochemical information processing method that can search for a reaction path of a bio-related compound and that can continuously display the reaction path. Within the method an apparatus is used which comprises input means that accept characteristic data about each atom constituting a compound and bonding pair data between atoms. This document also describes a method of generating a symbolic string in the form of canonical data which can uniquely specify a chemical structure of a compound on the basis of the characteristic data about each atom and the bonding pair data between atoms.

For operations requiring enormous trials such as searching the presence of a certain compound in any of existing databases and searching whether the same compound is stored in databases from different sources, it is convenient to have ID symbols as targets of matching instead of structural formulas of compounds. For that purpose, it is necessary to develop a method of assigning unique ID symbols defined uniquely to every structural formula of compounds.

Disclosure of Invention

An object of the present invention is to provide a means of assigning the same ID symbol to the same chemical structural formula whenever and wherever by providing a method of giving an ID symbol comprising a character string of fixed or variable length which is substantially unique with respect to a chemical structural formula of a compound. Another object of the present invention is to provide an index search method which enables us to employ a chemical structural formula directly as a query. As a result of zealous endeavor to solve the above-mentioned subjects, the inventors found that an ID symbol substantially unique to a chemical structural formula can be generated by carrying out a procedure of converting a chemical structural formula to a unique character string or a group of character strings. Moreover, the inventors succeeded in giving an ID symbol substantially unique to a chemical structural formula as a character string of adequate length of about 3 dozens of characters by combining a second procedure of converting the character string or the group of character strings to a shorter character string of fixed or variable length by a conversion function. The inventors also found that it is possible to perform a search equivalent to the case of the index search using a chemical structural formula as a query, by employing the above-mentioned ID symbol as a query of the index search. The present invention was achieved on the basis of the above-mentioned finings.

The present invention thus provides a method as specified in claim 1 of generating a character string of fixed or variable length which is substantially unique with respect to a chemical structural formula, based on the kind of each atom constituting the said chemical structural formula of a compound and the bonding relation between the atoms, and employing this character string as an ID symbol of the said compound. A numerical value is assigned to each atom depending on the element number of each atom and/or the kind of each atom constituting the chemical structural formula, the kind of isotope, or the kind of isomer arising from the atom, let the numerical value the first term of a number sequence, obtain number sequences by repeating operational rule defined by the covalent bond relation of the said group of atoms recurrently for limited number of times, generate a number sequence which is substantially unique to the said chemical structural formula obtained by arranging the number sequence by numerical comparison of each term, and a character string can be generated based on this number sequence.

Following the process in the above-mentioned method, a method including a process of converting the character string obtained by the above-mentioned process to a shorter character string of fixed or variable length by using a conversion function is also provided by the present invention. Collision intractable hash function and/or universal one-way hash function can be used as the conversion function, and preferably by using at least one function selected from message digest functions such as SHA, SHA1, MD-4, MD-5 and RIPEMD as the said conversion function; it is possible to generate a character string of fixed length, preferably comprising alphabets and/or arabic characters. To the character string or a group of character strings obtained by the above-mentioned method, one or more than one character strings concerning information which was not directly utilized (for example, one or more than one character strings concerning the kind of the method of generating the said ID symbol and/or the category information of the target of the said ID symbol) may be further added.

A method of the present invention can be carried out, preferably using a device comprising the following elements:

(a) A means of assigning a numerical value to each atom depending on the kind of each atom constituting a chemical structural formula;
(b) A means n which stores a vector with these values as elements;
(c) A means of inputting the covalent bond relation between the said atoms, and/or a storage means c which stores the relation as elements of a matrix;
(d) A storage means which stores number sequences generated by an operation formula using n and c, a device for the generation, and/or a medium which stores the operation procedure for the generation; and
(e) A medium which stores the result of obtaining a number sequence specific to the said chemical structural formula by rearranging the above-mentioned number sequences based on a numerical comparison rule and converting the said number sequence to a character string, a device which carries out the said conversion, and/or a medium which stores the conversion method

According to other preferred embodiment, the above-mentioned method can be carried out by using; a storage means n which stores vectors wherein a numerical value is assigned to each atom constituting the chemical structural formula as the kind of the atom, depending on the element number, the kind of isotope, and the kind of isomer arising from the atom, and these numbers are stored as elements of the vector; a storage means c which stores the covalent bond relation between the said atoms; a storage means n+1 which stores values obtained by adding to a vector element corresponding to each atom of the storage means n and/or the storage means n-1, the said vector element multiplied by a fixed number and/or the bond-order number, and vector elements corresponding to atoms, judged by using storage means c to be covalently bonded to the atom, multiplied by a fixed number and/or the bond-order number; a medium

which stores number sequences generated by rearranging, with every element or atom as a unit, elements of all vector sequence that are obtained by repeating these operations recurrently; and a device which outputs the number sequence as a unique character string for the chemical structural formula of the said compound.

From other aspect, an ID symbol unique to a chemical structural formula of a compound obtained by the above-mentioned method, and storage medium which stores the said ID symbol are provided. This ID symbol can be used for judging the identity or similarity of the chemical structural formulas of compounds. For example, it can be used to extract the information about identical or similar chemical structural formulas in one compound database or among more than one compound databases, and it can be used for the management of compound databases. For example, by appending the above-mentioned ID symbols to each file in a compound database or a database containing compound information, it is possible to search or match the compound information with ID symbols alone and without using the information on chemical structural formula.

The present invention further provides; a file, a record, a data object, a file name, a file path name, a record name, or a search key, which contains the above-mentioned ID symbol; a search query which contains the above-mentioned ID symbol and a device which generates the said query; a device for searching a file, a record, a data object, a file name, a file path name, or a record name, which contains the above-mentioned ID symbol; a medium which records a procedure for operating the above-mentioned device; the above-mentioned ID symbol which is used for the judgment of identity of the chemical structural formulas between compounds; the above-mentioned ID symbol which is used for the judgment of identity or similarity between compounds without using a part of or a whole chemical structural formula of a compound; a method of keeping confidentiality of the said chemical structural formula without comparing the chemical structural formula of the said compound directly by using the above-mentioned ID symbol; the above-mentioned method for giving a compatible ID symbol to the same compounds in more than one databases; and the above-mentioned ID symbol to be used for searching more than one databases with the same query. A file and a record are the same nature in the essence of the present invention, which is one mode of recording form of information in computer.

From other aspect, a storage medium which stores a computer program implementing the above-mentioned methods is provided by the present invention, and the above-mentioned device to carry out the above-mentioned methods and a medium which stores a program for operation of the said device are provided by the present invention. Any medium or device readable by a computer may be used as a storage medium, a storage means, a recording medium, and a recording device, and preferably, memory, flash memory, floppy disk, hard disk, CD-ROM, DVD, and MO can be used.

Brief Explanation of Drawing

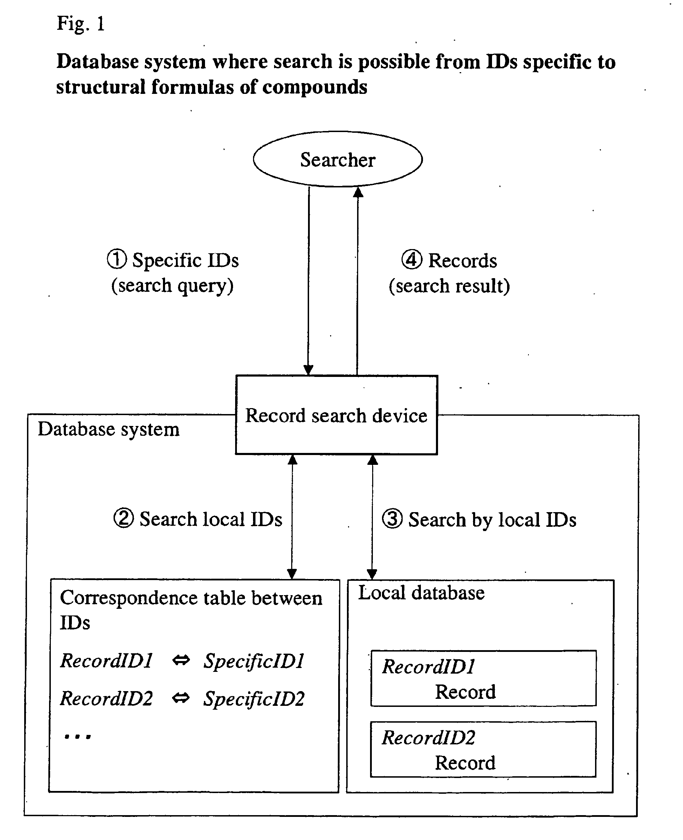

Fig. 1 is an example of the database system that can search records managed by local IDs by using specific IDs to chemical structural formulas of compounds as a query.

Best Mode to Carry out the Invention

Meanings of the terms used in the present description are as follows.

"Characters" are data which encode all or parts of character symbols used throughout the world, for example, alphabets, Arabic numerals, Japanese hiragana, Japanese katakana, Chinese characters, and the Hangul. "Character string" is a data which arranges more than one limited number of characters in order, and which is usually used by storing the said data in a computer-readable storage means. As a "character string", alphanumeric characters and data converted to bit strings by ASCII code are included. "Number sequence" is a data which arranges more than one limited number of rational numbers in order, and which is usually used by storing the said data in a compouter-readable storage means. Since data which are represented by a bit string of binary numbers are represented by 0 and 1, they may be interpreted as a character string or a number sequence.

As a storage device, it is preferred to use a device including computer memory that can store binary numbers, and in case of character strings, it is preferred to store them by converting each character to binary numbers of 1 to 4 byte length with a character code table. Although ASCII code or UNICODE is preferred as the character code table, any character code table is acceptable as long as a character and a number sequence is corresponded one by one. Moreover, when a group of multiple characters in a character string represents numerical values such as a decimal number or a hexadecimal number in a bunch, the value may be stored by converting it to a binary number, or those may be stored by converting each character to a binary number of 1 to 16 byte length by a character code table.

For a number sequence, it is preferred to store it by converting each rational number to a binary number of 1 to 8 byte length. In this case, a value of each rational number may be stored by converting to a binary number, or the value may be represented by a group of multiple characters as a decimal number or a hexadecimal number, and may be stored in the form similar to that for a character string. Data representing a character string or a number sequence in binary form are also called a "bit string" or "binary data" in this description.

"Chemical structural formula of a compound" generally means a figure which describes the kind of atoms,

bonding relation, the kind of bonds, the kind of isomers, and others in order for a chemist to describe a compound uniquely, however, it is used as a meaning of data that can specify a structure of a compound uniquely in a broader sense in this description. "Unique ID symbol" (sometimes called "specific ID symbol" in this description) means a property wherein ID symbols of the same compound are identical and ID symbols of compounds with different chemical structures are substantially nonidentical. In this description, "specific" instead of "unique" may be used to the meaning of the above-mentioned property. "Substantially nonidentical" means that it is not proved to be nonidentical logically, however, possibility of being identical is so small that it rarely happens to be identical in practical uses.

"Compound" is a group of atoms bonded together by covalent bonds, and includes inorganic compounds as well as organic compounds.

In the method of the present invention, a conversion procedure to represent a chemical structural formula of a compound with a unique character string is conducted. The kind of the method of converting a chemical structural formula to a uniform character string is not particularly limited so long as the same character string is always generated from the same chemical structural formula and substantially different character strings are generated from different chemical structural formulas. In the following, it is demonstrated using methanol ($CH_3OH$) as an example, however, conversion procedures applicable for the method of the present invention are not limited to the following.

"Generation of character string"

First, a means to store numerical values to be assigned to each atom is prepared. Although this storage means may be any means such as a register, a memory, a magnetic storage medium or a punch tape, a memory is most preferred as the computer-usable storage device. Numerical values defined by the "kind of atom" are assigned to these atoms. As the numerical values to be assigned, one can assign the element number of each atom, an arbitrarily defined numerical value depending on the kind of each atom, or different numerical values which even discriminate the kind of isotope. If the atom is an asymmetric center which causes optical isomers or it is related to cis bonding and trans bonding which causes geometrical isomers, one may assign numerical values that are defined so that the kind of isomers caused by the atom can be discriminated. Preferably, using a computer, data representing the chemical structural formula or the three-dimensional data of the said chemical structural formula which is the equivalent information to the chemical structural formula are input from an input means such as a file system, and the above-mentioned numerical values are automatically assigned to each atom. (In the following explanation, the element number is assigned for simplicity, however, the element number is not necessarily assigned in conversion process, and following procedure may be carried out by changing the value assignment in several ways. In the following drawing, assigned values are shown in parentheses. Also, a number in front of the atomic symbol is the order which distinguishes each atom for convenience.) The state wherein numerical values are assigned as initial values is called step 1.

Chemical formula (1)

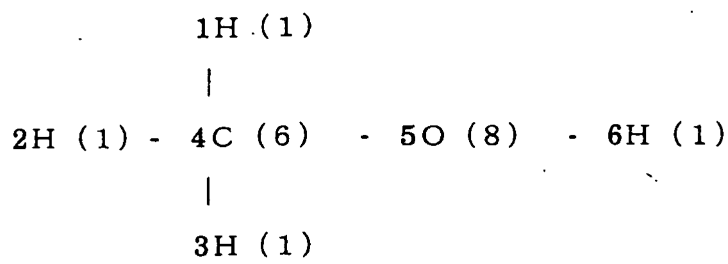

Numerical values assigned to each atom in step 1 are stored in storage means 1. Data stored in storage means 1 consists of multiple numerical values, and each numerical value is the numerical value assigned to each atom. If the number of atoms is M, it is possible to regard these multiple numerical values together (value 1, value 2, , , value M) as one M-dimensional vector. In this description, the said data is called a "vector", and a numerical value assigned to each atom in a vector is sometimes called an "element." Particularly, a vector stored in storage means 1 in step 1 is called "first term." In the case of chemical formula (1), the first term where numerical values are arranged in the order of 1H, 2H, 3H, 4C, 50, 6H is (1, 1, 1, 6, 8, 1) which is stored in storage means 1.

Furthermore, information representing covalent bond relation of atoms is stored in storage means c from the data representing the chemical structural formula that was input from an input means. As long as one can judge the atom to which an arbitrary atom is bonded covalently based on the data of storage means c, the data structure of storage

means c is not particularly limited. Preferably, when arbitrary pair of two atoms is bonded covalently, it returns true, if not, it returns false, a group of atoms is arranged in rows and columns, and use a matrix or a 2-dimensional array (c [1,2,,M][1,2,,,M]) storing 1 if the corresponding element of two atom pair is true, 0 if false, in electrical memory. In the case of chemical formula (1), since 5th 50 and 6th 6H are bonded, set c[5][6]=c[6][5]=1, and 4th 4C and 6th 6H are not bonded, therefore set c[4][6]=c[6][4]=0.

Next, storage means 2 same as the storage means 1 is prepared, and values newly assigned to each atom based on the value of each atom in step 1 as a result of the operational procedure explained below is stored in storage means 2 (called "step 2"). Furthermore, similar procedure is recurrently repeated and values assigned to atoms in each step are stored in storage means n (n=1,2,,,), (called "step n").

First, a value of each atom in step 2 is calculated as follows. A value of each atom in storage means 1 multiplied by a fixed number (preferably 1) is stored in storage means 2. Then, the number of partner atoms (partner for H is 1, 4 for C, 2 for O) with which each atom makes covalent bonds, examined by the storage means c, is multiplied by a fixed number (preferably 0), multiplied by the value of each atom in storage means 1, and added to the value of the said atom in storage means 2. Then, values in storage means 1 of the partner atoms to which each atom is bonded, examined by the storage means c, are multiplied by a fixed number (preferably 1) and added to the value of the said atom in storage means 2. The value of each atom in storage means 2 in step 2 carried out with preferable multipliers is shown below.

**Chemical formula (2)**

```
                    1H (1+6=7)
                     |
2H (1+6=7) - 4C (6+1+1+1+8=17) - 5O (8+6+1=15) - 6H (1+8=9)
                     |
                    3H (1+6=7)
```

Similarly, a value of each atom in step n is calculated as follows. A value of each atom in storage means k (wherein k is a group of n-or-less integers arbitrarily selected from integers that satisfy 1 ≦ k ≦ n, and preferably only one integer k = n is used) is multiplied by a fixed number (preferably 1) and stored in storage means n+1. Then, the number of partner atoms with which each atom makes covalent bonds, examined by the storage means c, is multiplied by a fixed number (preferably, 0 if n = 1, -1 if n > 1), multiplied by the value of each atom in storage means k (wherein k is a group of (n-1)-or-less integers selected from integers that satisfy 1 ≦ k ≦ n, and preferably only one integer k = n-1 is used only if n > 1), and is added to the value of the said atom in storage means n+1. Then, values in storage means k (wherein k is a group of n-or-less integers arbitrarily selected from integers that satisfy 1 ≦ k ≦ n, preferably only one integer k=n) of the partner atoms to which each atom is bonded, examined by the storage means c, are multiplied by a fixed number (preferably 1) and added to the value of the said atom in storage means n+1.

Number of steps may be repeated by arbitrary limited number of times (preferably about 10 times). This generates a vector sequence corresponding to each step. When it is processed by a computer, the above-mentioned operation rule is programmed as a recurrence formula, thus it is called a recurrence formula hereafter. The said recurrence formula is defined based on the information of covalent bonding between atoms which is stored in storage means c. For simplicity, the executed results to step 2 are explained here, however, the number of steps is not particularly limited in the embodiment of the present invention. Vectors in steps 1 and 2 are shown in the above-mentioned chemical formulas, the vector in storage means 1 is (1, 1, 1, 6, 8, 1), and the vector in storage means 2 is (7, 7, 7, 17, 15, 9). Here, the elements of the vectors arranged for each atom are as follows.

Chemical formula (3)

```
                1H (1, 7)
                  |
2H (1, 7) - 4C (6, 17) - 5O (8, 15) - 6H (1, 9)
                  |
                3H (1, 7)
```

Next, these elements are rearranged by a numerical comparison rule to generate a number sequence. For example, in an example where the value of each element is arranged in an ascending order, a number sequence of "1, 1, 1, 1, 7, 7, 7, 9, 15, 17" is generated as a "number sequence substantially unique to chemical structural formula." Furthermore, as other numerical comparison rule, it is possible to arrange them based on the comparison per atom, for example, the elements for each atom is compared with the value in storage means 1 first, and they are rearranged in an ascending order. If the values in storage means 1 are the same, the values in storage means 2 are compared and the order is obtained as follows. (1, 7), (1, 7), (1, 7), (1, 9), (6, 17), (8, 15)→1, 7, 1, 7, 1, 7, 1, 9, 6, 17, 8, 15. Then, this number sequence is converted to binary numbers as character code or numerical values, and a character string consisting of a bit string can be generated by arranging them in the order of the number sequence.

In this method, the more the number of the steps increases, the longer the whole character string becomes, and characteristics of the structure are emphasized. As for how many steps are necessary for the purpose of the present invention, the minimum number of steps is presumed by generating character strings for several tens of thousands of commercial compounds while varying the number of the steps, and by comparing the frequency of collision of the character strings (i.e. the same character string is generated from different structural formulas). Actual data were processed with this method and collisions of the character strings were examined, and consequently, it was confirmed that if we calculate with more steps to obtain longer character strings, collisions of character strings between compounds with different structures are avoided. When collisions of character strings were examined by generating number sequences with the present method for all compounds of ACD (a database of about 250,000 commercial compounds), collisions rarely occurred if the calculation was conducted to step 6. It is sufficient for actual compounds to set the number of steps to about 10 to ensure safety.

A character string thus generated is a character string of variable length which is substantially unique to a chemical structural formula. This character string is hereafter called a "structural character string". Since the structural character string is generated from the above-mentioned number sequence (sometimes called a "structural number sequence") and corresponds to a chemical structure one-by-one substantially, it can be used for the judgment of identity or similarity of chemical structures and can be used as an ID symbol. Moreover, each value in the number sequence may be represented as a character string by Arabic letters, those character strings may be connected by inserting an arbitrary punctuation character or a null character, and a character string thus composed as a whole may be used.

Although the element number is assigned to each atom as an initial numerical value in the above-mentioned explanation, an arbitrary numerical value may be assigned instead of the element number, the above-mentioned algorithm may be carried out to the final step with several conditions of initial value assignment, and number sequences obtained for each initial value may be bundled when composing a number sequence. For atoms having localized characteristics in the structure, it can be treated by changing the initial numerical value of the atom. For example, by changing the initial value of an atom concerning differences such as the geometrical isomerism and the steric isomerism, they can be reflected in the difference of the structural character string.

Although structural character string directly derived from the structural formula of a compound has various length, it is specific to the structural formula of the compound and generated from information of the structural formula alone, therefore, the structural character string itself may be used as an ID symbol for the judgment of identity or similarity as long as the obtained character string has length within an adequate range. If a shorter character string is used as an ID symbol, it is desirable to carry out a procedure by a conversion function. Using a conversion function even makes it possible to derive ID symbols of fixed length from structural character strings of varying length obtained above, therefore, the method including this process is a preferred embodiment of the present invention.

For example, a structural character string is converted to a bit string and stored in storage means b as explained below, an algorithm of converting to a bit string of short length of about 20 bytes is applied to storage means b, and the

converted bit string can be stored in storage means d. This may be converted to a character string, which may be output as a character-string ID symbol from output means. Here, as the storage means b and d, arbitrary devices may be used which can store binary numbers, but preferably, a register or a memory of a computer can be used. In the following explanation, an example wherein it is applied to the structural character string is explained for simplicity, however, it can be also applied to the structural number sequence in the same manner.

A character string as an ID symbol generated by the procedure with a conversion function should be specific to the structural character string, and is required to satisfy substantially the condition of the one-to-one mapping function as follows.

Same ID symbol should be generated from the same structural character string.

Different ID symbols should be generated from different structural character strings.

ID symbol should be a short character string of fixed length or variable length (preferably fixed length).

Generation method should be easy.

Examples of conversion functions preferably used in the method of the present invention are hash functions, more preferably, collision intractable hash functions and universal one-way hash functions. However, as the conversion functions used in the present invention, a strict mathematical proof is not necessarily for their collision intractability, preferably collision intractability and one-directionality, and practically, any function which gives conversion results satisfying the above-mentioned characteristics may be employed.

The universal one-way hash function was introduced by Naor and Yung, and is a function wherein it is difficult to obtain the value y which satisfies h(x) = h(y) when the function h and a value x in the defined area are given. On the other hand, the collision intractable hash function was introduced by Damgard, and is a function wherein it is difficult to obtain a pair of different values (x, y) which satisfies h(x) = h(y) when the function h is given.

As for the required conditions for the functions, the collision intractable hash function is more strict than the universal one-way hash function (for reviews of hash functions, consult T. Okamoto and H. Yamamoto, "Series / Johokagaku no Sugaku Gendai Ango (Mathematics of Information Science: Modern Cryptograph)", Sangyo Tosho; E. Okamoto, "Ango Riron Nyumon (Introduction to Encription Theory)", Kyoritsu Shuppan). In the present description, hash functions, particularly the collision intractable hash functions or the universal one-way hash functions, should be interpreted in the broadest sense, and in any sense, they should not be interpreted limitedly. For the methods of the present invention, any functions classified as collision intractable hash functions or universal one-way hash functions can be used.

Moreover, while aiming at having collision intractability, many hash functions have been proposed which were developed emphasizing the practical effectiveness. Since MD-4 and MD-5 by Rivest and PIPEMD and SHA (secure hash algorithm) which are based on MD-4 and MD-5 are widely used (Menezes, A. J., van Oorschot, P. and Vanstone, S. A.: Handbook of Applied Cryptography, CRC Press, 1996), these functions may be used for the methods of the present invention. Two or more different kinds of conversion functions, for example, two or more kinds of collision intractable hash functions, or two or more kinds of universal one-way hash functions may be used in combination. Furthermore, for example, one or more kinds of collision intractable functions and one or more kinds of universal one-way hash functions may be properly combined and used for processing. As a means of the present invention, it is particularly preferable to use SHA or SHA-1 solely, however, functions to be used and the combination of functions can be reasonably selected by those who are skilled in the art, in order to decrease the collision possibility of generated ID symbols thoroughly. In the present description, there are cases in which these functions are called message digest functions.

In the following, the algorithm of SHA used as a conversion function particularly favorably applicable to the method of the present invention is introduced. However, conversion functions applicable to the method of the present invention are not limited to SHA. In this example, the character strings generated by treatment with the hash functions as hash values are expressed by the combination of small-letter characters of alphabets and figures, but the characters are not limited to the small letters of alphabets. Characters used for the method of the present invention may be either capital letters or small letters, capital letters and small letters may be used without distinction, or they may be used with distinction. Furthermore, more rapid hash method with higher collision intractability may be used as a substitute for SHA. For example, SHA1 which is an improved version of SHA may be used.

*Input data

Bit string [m] of an arbitrary length which is less than $2^{64}$ bits (a method of converting structural character string data to a bit string [m] is described later).

*Output data

Hash values of 160 bits are generated to [m].
Padding is conducted as in the following procedures in order for the input bit string [m] to be multiple numbers of 512 bits (16 x 32 bits)

Procedure 1) A bit string of "100 · · · 00" is added to the end of [m] in order for the bit string length of [m] to be '512N-64'.

Procedure 2) Bit-string length of the input string is expressed by 64 bits, and further added to the end of the bit string.

The padded bit string is divided into N blocks of 512 bits each, which forms $M_1$, $M_2$, · · ·, Mn.

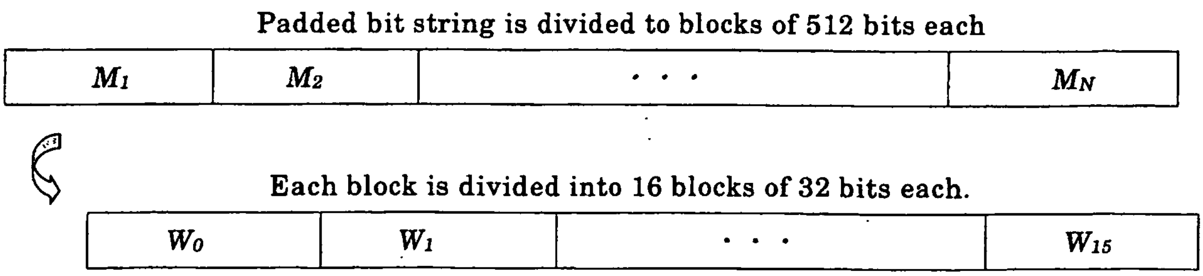


To the above-mentioned bit string, hash values are calculated by the following procedures using the following constants and functions.

Constants)

The following constants are represented by hexadecimal numbers.

H0 = 67452301
H1 = EFCDAB89
H2 = 98BADCFE
H3 = 10325476
H4 = C3D2E1F0
Kt = 5A827999 ($0 \leqq t \leqq 19$)
Kt = 6ED9EBA1 ($20 \leqq t \leqq 39$)
Kt = 8F1BBCDC ($40 \leqq t \leqq 59$)
Kt = CA62C1D6 ($60 \leqq t \leqq 79$)

Functions)

$$ft(x, y, z) = (x \wedge y) \vee (x \wedge z) \qquad (0 \leqq t \leqq 19)$$

$$ft(x, y, z) = x \oplus y \oplus z \qquad (20 \leqq t \leqq 39)$$

$$ft(x, y, z) = (x \wedge y) \vee (x \wedge z) \vee (y \wedge z) \qquad (40 \leqq t \leqq 59)$$

$$ft(x, y, z) = x \oplus y \oplus z \qquad (60 \leqq t \leqq 79)$$

Note)

$\wedge$ : Logical product (AND)
$\vee$ : Logical sum (OR)
$\oplus$ : Exclusive logical sum (XOR)

* Procedure

The following process is repeated for i equals 0 to N.

(1) *Mi* is divided into 16 blocks of 32 bits each, which are labeled $W_0, W_1, \cdots, W_{15}$, where $W_0$ is the left end block.
(2) $W_t = (W_{t-3} \oplus W_{t-8} \oplus W_{t-14} \oplus W_{t-16})$ <<< 1 (only in the case of SHA-1)
determines $W_{16}, \cdots, W_{79}$ (32 bits each)
rf.4) "X <<< n" means a rotation shift of bit string to X by n bits to the left.
Rotation Shift: bit string is slid by one bit in certain direction, and the character at one end is moved to the opposite end.

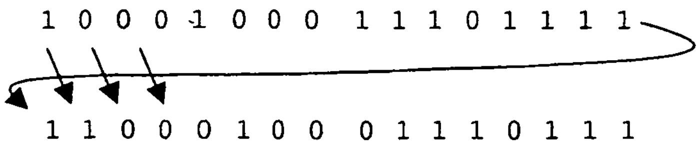


(3) The variables A, B, C, D, E are initialized as follows. A = H0, B = H1, C = H2, D = H3, E = H4 (32 bits each)
(4) The following steps are repeated for t equals 0 to 79. rf.5) '+' indicates the remainder which is obtained by the division of the sum of the values on the left side and right side by '$2^{32}$'.

$$TEMP = (A <<< 5) + ft(B, C, D) + E + W_t + Kt$$

E = D

D = C

C = B <<< 30

B = A

A = TEMP

(5) H0 = H0 + A, H1 = H1 + B, H2 = H2 + C, H3 = H3 + D, H4 = H4 + E

The total of 160 bits combined by the bit string of H0 ~ H4 finally obtained become hash values.
A method of converting a structural character string to a bit string is explained in the following. By ASCII code, generate a bit string by converting each character to 8 bits and arrange them in the order of the character string. Although this procedure is employed in the examples of the present description, conversion code other than ASCII code may be used when a character code is converted to a bit string. Here, "bit string" means a series of arranged 1 bit information. One bit corresponds to one place of a binary number and represented by 0 or 1. When ASCII code is employed, a condition of SHA which allows less than $2^{64}$ bits means a number of characters less than about 2 x $10^{18}$, therefore, a structural character string with fairly high steps can be described.
In order to utilize 160-bit string obtained by the above-mentioned hash function as an ID, they are divided to necessary number of bits so that the bit string can be represented by alphanumeric characters. 160 bits are divided to 5 bits each, and each 5 bits are represented with a radix of 32 using 32 characters of 0, 1, 2, 3, 4, 5, 6, 7, 8, 9, a, b, c,

d, e, f, g, h, i, j, k, l, m, n, o, p, q, r, s, t, u, v.

The following 160-bit string is divided to 5 bits each, and converted to corresponding characters with a radix of 32,

```
10000110 11110111 11100100 00110111··· 10111000

                    ⇩

  10000    11011    11011    11110    01000 ···
    |        |        |        |        |
    g        r        r        u        8
```

and after the conversion, we get an ID as follows.

"g r r u 8 d v q k m j v p o a t 3 n e b j q n a t 8 r n c p t o"

To the ID symbol generated by the method of the present invention, one or more character strings of fixed length, preferably consisting of alphanumeric characters, may be further appended to crate a new ID symbol, in order to add information such as the information indicating the kind of the corresponding data (for example, information indicating that the ID symbol refers to a compound) and the information indicating the kind of generation method of the ID symbol (for example, information indicating the kind of the hash function used). Character strings to be added may be placed at any part, for example, to the head or tail of the character string obtained by application of the hash function. Preferably, one character string is added to the head of the character string obtained as a hash value.

ID symbols of the present invention may be used for the management and matching of compound data (including chemical structural formula data). Since the above-mentioned ID symbol is specific to each compound and the possibility of collision is very low, it is possible to judge simply and rapidly the identity of compounds by processing multiple compounds with the method of the present invention, generating ID symbols, and comparing the ID symbols. For example, using the above-mentioned ID symbols, it is possible to search rapidly the chemical structural formula identical to a specific compound from a compound database. Furthermore, using the above-mentioned ID symbols, it is possible to manage compound databases. For example, it is possible to generate the above-mentioned ID symbols for compounds in databases, and to detect compounds included redundantly in a compound database, or to detect compounds redundantly registered in different compound databases simply and rapidly. Moreover, when new compound information is registered to a database, it becomes possible to search simply whether the compound is already registered or not. Furthermore, it is possible to keep confidentiality of compound data by disclosing only ID symbols for the comparison of compounds, without disclosing the compound data themselves.

Whereas the method of the present invention is convenient for the purpose of searching and matching basically the same chemical structural formula, the method can be also used for the purpose of classification by detecting similar chemical structural formulas such as derivatives. Moreover, it can be used for the purpose of detecting a compound with a similar chemical structural formula by doing as follows. For a certain chemical structural formula, it is preferable to store ID symbols generated for (not necessarily one) structures removed with substituents, in addition to the ID symbol of the structural formula itself. For example, if an ID symbol generated by substituting C1 with H for a chlorinated compound and an ID symbol generated by substituting Br with H for a brominated compound coincides, it is possible to judge automatically that they are the homologous compounds, and it can be operated similarly for compounds with more complex structural formula. There is no limitation to the number of the ID symbols, and ID symbols generated from the original structure are stored in order. If multiple ID symbols are generated and stored similarly for all compounds in databases, it is possible to examine rapidly, between databases of different origin, whether there are compounds of a certain derivative family or whether there are derivatives for a specific compound in the compound databases. It should be understood that such embodiments are also included in the scope of the present invention.

## Examples

In the following, the present invention is explained in more details with examples, however, the scope of the present invention is not limited to the following examples.

Example 1

The above-mentioned method was applied to ethanol and dimethyl ether whose composition formula is $C_2H_6O_1$ (the method referred to as "preferred" was employed here).

Ethanol

Step 1)

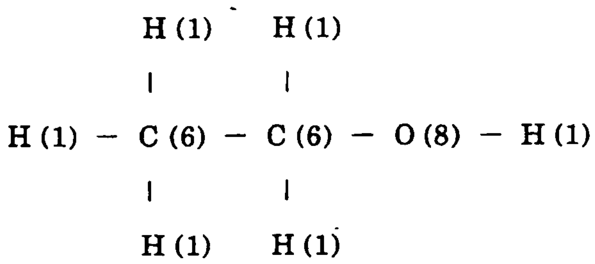

Step 2)

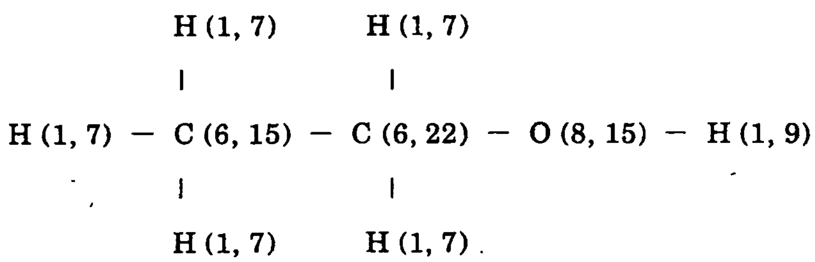

Structural number sequence:
1,7,1,7,1,7,1,7,1,7,1,9,6,15,6,22,8,15
Structural character string:
" 1 7 1 7 1 7 1 7 1 7 1 9 6 15 6 22 8 15"
ID : cb6mv472bodkdaelhsgvrda77dlvlhgr

Dimethyl ether

Step 1)

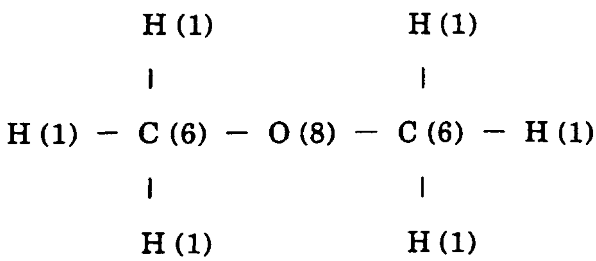

Step 2)

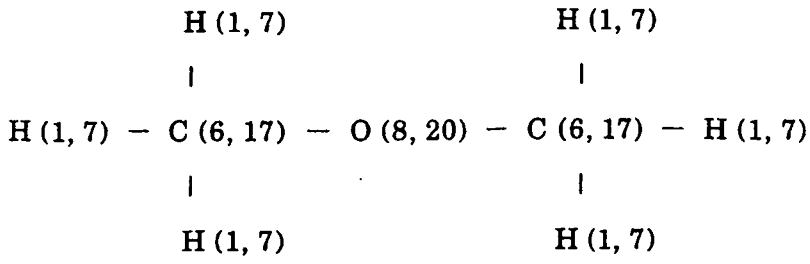

Structural number sequence
1,7,1,7,1,7,1,7,1,7,1,7,6,17,6,17,8,20
Structural character string
"1 7 1 7 1 7 1 7 1 7 1 7 6 17 6 17 8 20"
ID : 9o7fkpmigj73svgq6gqist2sjuillshn

Thus, different structural number sequences, structural character strings, and IDs were obtained for ethanol and dimethyl ether.

Example 2

An example of the construction of a database system which eneables searching of records using specific IDs to chemical structural formulas of compounds as a query is given in Fig.1.

In the database system, an ID is given to a record and is managed internally. The IDs of records (*RecordID1, RecordID2*, etc. in Fig.1) are used locally inside this database system, therefore called local IDs here. In the database system, there is a correspondence table for specific IDs of structural formulas of compounds and local IDs, and this correspondence can be altered, added, or deleted by an administrator of the database system. Furthermore, there exists in the database system a local database which manages records with local IDs, therefore, it is possible to search records with the local IDs. The record search device, the correspondence table between IDs, and the local databases may exist at physically separated places, and the communication between them may be conducted via internet or intranet. Moreover, the administrator for the correspondence table between IDs and the administrator for the local databases may be different. Any means may be used for the correspondence table between IDs as long as corresponding local IDs can be searched from the specific IDs. The correspondence between specific IDs and local

IDs may be multiple to multiple.

The procedure for search is the following. A searcher outside the database system transmits a search query containing one or more specific IDs to a structural formula of a compound to a record search device of the said database system (Fig. 1①). The record search device searches local IDs corresponded with the said specific IDs from the correspondence table between IDs (Fig: 1②). Next, using the said local IDs, the record search device searches records with the said local IDs from the local database (Fig. 1③). Finally, the record search device transmits the said records back to the searcher.

As other method, if the correspondence table between IDs and tables of local databases are merged in advance using a view generating function of the relational database, it is possible to perform searching of ② and ③ together.

In this example, a searcher can search databases only with specific IDs to structural formulas of compounds, and furthermore, at the time of search, the searcher can search records with local IDs corresponded by the "correspondence table between IDs." The database system administrator can set the system to always transmit an appropriate record to the structural formula of a compound requested by the searcher, by altering the correspondence between specific IDs and local IDs appropriately when records in the local databases have been altered, added, or deleted. Moreover, when a record to which a specific ID corresponds ceases to exist in the local database by alteration or deletion of the record, the database system administrator can set the system to transmit correction information about it back to the searcher, instead of the record. Furthermore, a searcher can search multiple databases at the same time by transmitting the same specific ID as a query simultaneously to multiple database systems shown in Fig. 1 via internet or intranet. Moreover, if records of the present system are limited to files containing specific IDs, it is convenient to use a commercially-available index search program. In this case, the index search program automatically recognizes specific IDs in the file as keywords, and generates correspondences between the said IDs and paths of the said files (this is

equivalent to the correspondence table between IDs). Thus, by transmitting a specific ID to the index search program, it is possible to search a file containing the said specific ID.

Industrial Applicability

By the method of the present invention, it is possible to generate, to a compound with any structure, a unique ID symbol to the chemical structural formula of the compound as a character string or a group of character strings of fixed or variable length with extreme low probability of collision. This ID symbol can be generated extremely rapidly and easily from the chemical structural formula of a compound, and since the ID symbol is specific to the chemical structural formula of each organic compound and possibility of collision is practically very rare, identity or similarity of chemical structural formulas can be judged easily by comparing the ID symbols only. The ID symbol can be used for database management so that there is no redundancy in entries, can make it possible to use compound databases generated at different sites unitary, and also to examine rapidly whether a certain compound or its derivatives exist in the compound databases or not.

Furthermore, since the ID symbol of the present invention is generated from the chemical structural formula itself by a software, as long as the said software is distributed, the same ID symbol is given to the same structure throughout the world, and can be used for database searching and matching instead of the chemical structural formula. Since there is no need to search databases using the chemical structural formula itself as a query, leakage of confidentiality to outside at the time of transmission or search can be avoided. Moreover, if a database administrator adds the ID symbols to all compounds in databases, they can be used for avoiding the redundancy and for linking between different databases of different origins. Furthermore, for the chemical structural formula of a compound that a researcher have synthesized or is going to synthesize, the ID symbol can be given easily with the same software, and it is possible to perform database search and matching of structures.

## Claims

**1.** A computer implemented method of generating a character string of fixed or variable length which is substantially unique to a chemical structural formula based on the kind of each atom constituting the said chemical structural formula and the bonding relation between the said atoms, and employing the character string as an ID symbol identifying the said chemical structural formula and/or a compound specified by the said chemical structural formula, **characterized in that** the character string is generated by the following steps:

(1) A first vector whose element is a numerical value defined for the kind of element of each atom constituting said chemical structural formula is set as the first term,
(2) A recurrence formula is defined wherein a value of each atom in the next vector is determined by adding a value of each atom in the current vector multiplied by a fixed number, the number of partner atoms with which each atom makes covalent bonds multiplied by a fixed number and multiplied by the value of each atom in the current vector or in a previous vector, and values of the partner atoms in the current vector or in a previous vector to which each atom is bonded multiplied by a fixed number,
(3) A limited number of vector sequence is generated based on the said first term and the said recurrence formula,
(4) By rearranging all elements in all vectors by a numerical comparison rule, a "number sequence substantially unique to said chemical structural formula" is generated, wherein the same chemical structural formula gives the same number sequence.
(5) Said character string is generated by converting each term of the said number sequence in the order of the said number sequence.

**2.** The method according to claim 1, wherein a vector whose element is a numerical value defined for the kind of element of each atom constituting said chemical structural formula and for the kind of isomers arising from the said atom is used as the first term.

**3.** A method of claim 1 or 2 further comprising converting the generated character string to a character string of fixed length by a conversion function which is substantially a one-to-one mapping function, and employing the converted character string as a symbol identifying said chemical structural formula.

**4.** The method according to claim 3, wherein a collision intractable hash function and/or universal one-way hash function is employed as a conversion function which is substantially a one-to-one mapping function.

5. The method according to claim 3 or claim 4, wherein a message digest function is employed as a conversion function which is substantially a one-to-one mapping function.

6. The method according to any one of claims 1 through 5, comprising the step of adding one or more character strings with category information representative of the generation method of the said ID symbol and/or the target of the said ID symbol.

7. The method according to any one of claims 1 through 6, further comprising generating ID symbols of multiple chemical structural formulas and judging the identity of said formulas by comparing said ID symbols only.

8. The method according to claim 7, wherein confidentiality of the chemical structural formulas is maintained by employing said ID symbols and wherein said IDsymbols are generated according to claim 4 or 5.

9. The method according to any one of claims 1 through 6, further comprising identifying the same chemical structural formula by an identical ID symbol in more than one database.

10. The method according to any one of claims 1 to 6 further comprising searching files using the generated ID symbol.

11. An apparatus comprising means specially adapted to carry out all the steps of any one of claims 1 to 6.

12. A computer program comprising code means for carrying out all the steps of any one of claims 1 to 6.

13. A medium storing ID symbols generated according to the method of any one of claims 1 to 6.

**Patentansprüche**

1. Computergestütztes Verfahren zur Erzeugung einer Zeichenkette fester oder variabler Länge, die im Wesentlichen einzigartig für eine chemische Strukturformel ist, und zwar auf Grundlage der Art eines jeden am Aufbau der chemischen Strukturformel beteiligten Atoms und der Bindungsbeziehung zwischen den Atomen, und zur Verwendung der Zeichenkette als ID-Symbol, das die chemische Strukturformel und/oder eine Verbindung identifiziert, die durch die chemische Strukturformel angegeben ist, **dadurch gekennzeichnet, dass** die Zeichenkette durch die folgenden Schritte erzeugt wird:

   (1) ein erster Vektor, dessen Element ein numerischer Wert ist, der für die Elementsorte eines jeden am Aufbau der chemischen Strukturformel beteiligten Atoms definiert ist, wird als erster Term festgelegt;
   (2) es wird eine Rekursionsformel definiert, wobei ein Wert eines jeden Atoms im nächsten Vektor bestimmt wird durch Addieren eines Werts eines jeden Atoms im gegenwärtigen Vektor, multipliziert mit einer festen Zahl, die Zahl der Partneratome, mit denen jedes Atom covalente Bindungen eingeht, multipliziert mit einer festen Zahl und multipliziert mit dem Wert eines jeden Atoms im gegenwärtigen oder in einem vorhergehenden Vektor, und Werte der Partneratome im gegenwärtigen oder in einem vorhergehenden Vektor, an das jedes Atom gebunden ist, multipliziert mit einer festen Zahl;
   (3) es wird eine begrenzte Vektorsequenzzahl auf der Grundlage von erstem Term und Rekursionsformel erzeugt;
   (4) durch Umformen aller Elemente in allen Vektoren mit Hilfe einer numerischen Vergleichsregel wird eine "Zahlenfolge, die im Wesentlichen einzigartig für die chemische Strukturformel ist" erzeugt, wobei die gleiche chemische Strukturformel die gleiche Zahlenfolge ergibt;
   (5) die Zeichenkette wird erzeugt durch Umrechnen eines jeden Terms dieser Zahlenfolge in der Reihenfolge der Zahlenfolge.

2. Verfahren nach Anspruch 1, wobei ein Vektor, dessen Element ein numerischer Wert ist, der für die Art des Elements eines jeden am Aufbau der chemischen Strukturformel beteiligten Atoms und für die sich aus dem Atom ergebende Art von Isomer definiert ist, als erster Term verwendet wird.

3. Verfahren nach Anspruch 1 oder 2, des Weiteren umfassend die Umrechnung der erzeugten Zeichenkette in eine Zeichenkette fester Länge mit Hilfe einer Umrechnungsfunktion, die im Wesentlichen eine Eins-zu-eins-Mapping-Funktion ist, und die Verwendung der umgerechneten Zeichenkette als Symbol, das die chemische Strukturformel angibt.

4. Verfahren nach Anspruch 3, wobei eine kollisionsfreie Hashfunktion und/oder eine universelle Einweg-Hashfunktion als Umrechungsfunktion verwendet wird, bei der es sich im Wesentlichen um eine Eins-zu-eins-Mapping-Funktion handelt.

5. Verfahren nach Anspruch 3 oder 4, wobei eine Message Digest-Funktion als Umrechnungsfunktion verwendet wird, die im Wesentlichen eine Eins-zueins-Mapping-Funktion ist.

6. Verfahren nach einem der Ansprüche 1 bis 5, umfassend den Schritt des Addierens einer oder mehrerer Zeichenketten mit Kategorieinformation, die das Generierungsverfahren dieses ID-Symbols und/oder des Ziels dieses ID-Symbols angibt.

7. Verfahren nach einem der Ansprüche 1 bis 6, des Weiteren umfassend die Erzeugung von ID-Symbolen mehrerer chemischer Strukturformeln und die Beurteilung der Identität der Formeln durch Vergleichen nur dieser ID-Symbole.

8. Verfahren nach Anspruch 7, wobei die Vertraulichkeit der chemischen Strukturformeln durch Verwendung der ID-Symbole aufrechterhalten wird und wobei diese ID-Symbole nach Anspruch 4 oder 5 erzeugt werden.

9. Verfahren nach einem der Ansprüche 1 bis 6, des Weiteren umfassend die Identifizierung der gleichen chemischen Strukturformel durch ein identisches ID-Symbol in mehr als einer Datenbank.

10. Verfahren nach einem der Ansprüche 1 bis 6, des Weiteren umfassend die Suche nach den Dateien, bei denen das erzeugte ID-Symbol verwendet wird.

11. Vorrichtung, umfassend Hilfsmittel, die speziell zur Durchführung sämtlicher Schritte nach einem der Ansprüche 1 bis 6 angepasst sind.

12. Computerprogramm, umfassend Codierungshilfsmittel für die Durchführung sämtlicher Schritte nach einem der Ansprüche 1 bis 6.

13. Medium, das ID-Symbole abspeichert, die gemäß dem Verfahren nach einem der Ansprüche 1 bis 6 erzeugt wurden.

**Revendications**

1. Procédé informatique permettant de générer une chaîne de caractères de longueur fixe ou variable qui est fondamentalement propre à une formule de structure de composé fondée sur le type de chaque atome constituant ladite formule de structure de composé et les relations de liaison entre lesdits atomes, et employant la chaîne de caractère comme empreinte de ladite formule de structure de composé et/ou d'un composé spécifié par ladite formule de structure de composé, **caractérisé en ce que** la chaîne de caractère est générée par les étapes suivantes :

   (1) un premier vecteur dont l'élément est une valeur numérique définie pour le type d'élément de chaque atome constituant ladite formule de structure de composé est posé comme premier terme,
   (2) une formule de récurrence est définie, dans laquelle une valeur de chaque atome dans le vecteur suivant est déterminée en ajoutant une valeur de chaque atome dans le vecteur courant multipliée par un nombre fixe, le nombre d'atomes partenaires avec lequel chaque atome est engagé dans des liaisons covalentes multiplié par un nombre fixe et multiplié par la valeur de chaque atome dans le vecteur courant ou dans un vecteur précédent, et les valeurs des atomes partenaires dans le vecteur courant ou dans un vecteur précédent auquel chaque atome est lié multiplié par un nombre fixe,
   (3) un nombre limité de séquence de vecteur est généré sur la base dudit premier terme et de ladite formule de récurrence,
   (4) en réarrangeant tous les éléments dans tous les vecteurs par une règle de comparaison numérique, un « numéro de séquence fondamentalement propre à ladite formule de structure de composé » est généré dans lequel la même formule de structure de composé donne le même numéro de séquence,
   (5) ladite chaîne de caractère est générée en convertissant chaque terme dudit numéro de séquence dans l'ordre dudit numéro de séquence.

2. Procédé selon la revendication 1, dans lequel un vecteur dont l'élément est une valeur numérique définie pour le type d'élément de chaque atome constituant ladite formule de structure de composé et pour le type d'isomères

émanant dudit atome est utilisé comme premier terme.

3. Procédé selon la revendication 1 ou 2, comprenant en outre la conversion de la chaîne de caractère générée en une chaîne de caractère de longueur fixe par une fonction de conversion étant essentiellement une fonction de projection un pour un, et employant la chaîne de caractères convertie comme empreinte de ladite formule de structure de composé.

4. Procédé selon la revendication 3, dans lequel une fonction de hachage résistante aux collisions et/ou une fonction de hachage universelle à sens unique est employée comme fonction de conversion qui est essentiellement une fonction de projection un pour un.

5. Procédé selon la revendication 3 ou 4, dans lequel une fonction de condensation de données est employée comme fonction de conversion laquelle est essentiellement une fonction de projection un pour un.

6. Procédé selon l'une quelconque des revendications 1 à 5, comprenant l'étape d'ajouter une chaîne de caractère ou plus avec des informations sur la catégorie représentatives de la méthode de génération de ladite empreinte et/ou de la cible de ladite empreinte.

7. Procédé selon l'une quelconque des revendications 1 à 6, comprenant en outre la génération des empreintes de multiples formules de structures de composés et jugeant l'identité desdites formules uniquement par comparaison desdites empreintes.

8. Procédé selon la revendication 7, dans lequel la confidentialité des formules de structures de composés est préservée en employant lesdites empreintes et dans lesquels lesdites empreintes sont générées selon la revendication 4 ou 5.

9. Procédé selon l'une quelconque des revendications 1 à 6, comprenant en outre l'identification de la même formule de structure de composé par une empreinte identique dans plus d'une base de données.

10. Procédé selon l'une quelconque des revendications 1 à 6, comprenant en outre la recherche de fichiers en utilisant l'empreinte générée.

11. Appareil comprenant des moyens spécialement adaptés pour mener à bien toutes les étapes de l'une quelconque des revendications 1 à 6.

12. Programme informatique comprenant du code permettant de réaliser toutes les étapes de l'une quelconque des revendications 1 à 6.

13. Média stockant les empreintes générées selon le procédé de l'une quelconque des revendications 1 à 6.

Fig. 1

**Database system where search is possible from IDs specific to structural formulas of compounds**